(19) Europäisches Patentamt
European Patent Office
Office européen des brevets

(11) EP 2 748 153 B1

(12) # FASCICULE DE BREVET EUROPEEN

(45) Date de publication et mention de la délivrance du brevet:
**14.12.2016 Bulletin 2016/50**

(21) Numéro de dépôt: **12750773.9**

(22) Date de dépôt: **24.08.2012**

(51) Int Cl.:
***C07D 307/50*** *(2006.01)*

(86) Numéro de dépôt international:
**PCT/EP2012/066545**

(87) Numéro de publication internationale:
**WO 2013/030131 (07.03.2013 Gazette 2013/10)**

(54) **PROCEDE DE PREPARATION DE FURFURAL**

VERFAHREN ZUR HERSTELLUNG VON FURFURAL

METHOD FOR PREPARING FURFURAL

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **26.08.2011 FR 1157563**

(43) Date de publication de la demande:
**02.07.2014 Bulletin 2014/27**

(73) Titulaires:
- **Centre National de la Recherche Scientifique (C.N.R.S.)
75016 Paris (FR)**
- **UNIVERSITE CLAUDE BERNARD - LYON 1
69100 Villeurbanne (FR)**

(72) Inventeurs:
- **ESSAYEM, Nadine
F-38540 Saint Just Chaleyssin (FR)**
- **LOPES DE SOUZA, Rodrigo
CEP- 21050-650 Rio de Janeiro RJ (BR)**
- **RATABOUL, Franck
F-69008 Lyon (FR)**
- **DOISEAU, Aude-Claire
F-69006 Lyon (FR)**

(74) Mandataire: **Lavoix
62, rue de Bonnel
69448 Lyon Cedex 03 (FR)**

(56) Documents cités:
**EP-A1- 0 111 699 WO-A1-02/053829
WO-A1-2009/130386 WO-A1-2011/161141
US-A- 2 999 783**

- **SHARMA D K ET AL: "Elevated temperature hydrolysis of rice husk with pressurized water in a semibatch process", CELLULOSE CHEMISTRY AND TECHNOLOGY, EDITURA ACADEMIEI ROMANE, RO, vol. 17, no. 6, 1 janvier 1983 (1983-01-01), pages 655-658, XP009160598, ISSN: 0576-9787**
- **AMAR SINGH ET AL: "Integrated process for production of xylose, furfural, and glucose from bagasse by two-step acid hydrolysis", INDUSTRIAL & ENGINEERING CHEMISTRY PRODUCT RESEARCH AND DEVELOPMENT, vol. 23, no. 2, 1 juin 1984 (1984-06-01), pages 257-262, XP055026427, ISSN: 0196-4321, DOI: 10.1021/i300014a017**
- **AJIT SINGH MAMMAN ET AL: "Furfural: Hemicellulose/xylosederived biochemical", BIOFUELS, BIOPRODUCTS AND BIOREFINING, vol. 2, no. 5, 1 septembre 2008 (2008-09-01), pages 438-454, XP055031375, ISSN: 1932-104X, DOI: 10.1002/bbb.95**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 748 153 B1

## Description

La présente invention concerne la préparation de furfural

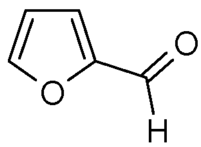

par déshydratation de xylose.

Le furfural est un produit issu de la dégradation de la biomasse. Le furfural est notamment utilisé dans le domaine pétrochimique, par exemple pour le raffinage. Le furfural, ainsi que son dérivé l'alcool furfurylique, peut être utilisé seul ou en association avec le phénol, l'acétone, ou l'urée pour fabriquer des résines furaniques. Le furfural est également utilisé comme intermédiaire de synthèse pour la production de solvants comme les furanes et le tétrahydrofuranne.

Actuellement le furfural est essentiellement produit en Chine via des procédés discontinus appelés « Quaker Oats ». Ce procédé consiste en une déshydratation du xylose dans un milieu acide sulfurique. Cependant, ce procédé consomme beaucoup d'énergie pour un rendement faible en furfural (30-35%).

Les déshydratations menées en milieu aqueux ne sont pas sélectives. Le manque de sélectivité de la réaction de déshydratation des pentoses en furfural s'explique par la rapidité des réactions secondaires de polymérisation des intermédiaires réactionnels ou du furfural en milieu aqueux (par exemple formation d'humine). Différents procédés ont été développés pour tenter d'améliorer la conversion des pentoses et la sélectivité en furfural.

On connaît de WO2007/146636 un procédé permettant la préparation de furfural à partir de xylose en milieu biphasique comprenant une phase aqueuse acide formée d'eau, de DMSO et d'HCl en tant que catalyseur, et une phase organique comprenant du MIBK et du 2-butanol. Le furfural produit dans la phase aqueuse est extrait à l'aide du solvant organique. Ce document décrit de manière plus générale la déshydratation d'hydrates de carbone en dérivés de furane, dans un milieu biphasique et en présence préférentiellement d'un acide minéral en tant que catalyseur homogène.

On connaît également de Moreau et al (Industrial Crops and Products, 1998, 7, 95-99) la déshydratation du xylose en furfural dans un mélange eau/toluène en présence de H-Y faujasites et H-modenites. On connaît également de Lima et al (Applied Catalysis A : General 388, 2010, 141-148) la déshydratation du xylose en furfural dans un mélange eau/toluène en présence de zéolite. On connaît enfin de Wang et al (Energies, 2011, 670-684) la déshydratation du xylose en furfural dans un mélange eau/toluène en présence de catalyseurs mésoporeux fonctionnalisés par des groupements acide sulfonique. Cependant, ce type de procédé nécessite l'emploi de solvants pouvant être toxiques, nécessitant donc des purifications du furfural obtenu, et dont les températures d'ébullition sont élevées, ce qui fait de ces procédés des procédés complexes et coûteux.

On connaît de Hubert et al (Journal of Catalysis, 2011, 279, 174-182) l'utilisation de catalyseur acide solide pour la déshydratation du xylose en furfural dans l'eau. On connaît également de Hubert et al (Green Chemistry, 2010, 12, 1423-1429) la déshydratation du xylose en furfural en solution aqueuse contenant du HCl ou dans un milieu biphasique eau/solvant organique par chauffage micro-onde.

Ainsi, afin d'augmenter la conversion en pentoses et la sélectivité en furfural, des techniques de plus en plus complexes ont été développées, sans pour autant atteindre les rendements espérés.

La production de furfural d'une façon sélective est complexe et sa purification est difficile en raison de l'instabilité de cette molécule.

Il y a donc un intérêt à fournir un procédé de préparation de furfural qui réponde aux inconvénients des procédés de l'état de la technique.

Un objectif de la présente invention est de fournir un procédé de préparation de furfural avantageux d'un point de vue industriel.

Un autre objectif de la présente invention est de fournir un procédé de préparation de furfural avec une sélectivité et une conversion élevées.

Un autre objectif encore de la présente invention est de fournir un procédé de préparation de furfural permettant de réduire, voire de supprimer, la formation de produits secondaires, notamment de type humine.

Un objectif de l'invention est également de fournir un procédé pouvant être conduit en milieu aqueux et sans nécessiter des solvants organiques potentiellement toxiques, avec ou sans catalyseurs.

D'autres objectifs apparaîtront à la lecture de la description de l'invention qui suit.

Tous ces objectifs sont remplis par l'invention qui concerne un procédé de préparation de furfural par réaction de xylose dans l'eau et en présence d'acide carboxylique, dans une solution aqueuse d'acide carboxylique comme milieu réactionnel, en présence d'un catalyseur hétérogène. De façon avantageuse, le procédé de l'invention permet

d'obtenir des conversions en xylose supérieures à 50 %, de préférence supérieures à 90 %. De façon avantageuse, le procédé de l'invention permet d'obtenir une sélectivité en furfural supérieure à 50 %, de préférence supérieure à 75 %.

Les acides carboxyliques peuvent être des monoacides, des diacides ou des triacides. Ils sont notamment choisis parmi :

- les acides de formule R-COOH dans laquelle R représente un atome d'hydrogène ou une chaîne alkyle, linéaire ou ramifiée, en $C_1$ à $C_5$, de préférence en $C_1$ à $C_3$, éventuellement substituée par un ou plusieurs groupes OH ;
- les acides de formule HOOC-L-COOH dans laquelle L représente une liaison ou une chaîne alkyle, linéaire ou ramifiée, en $C_1$ à $C_5$, de préférence en $C_1$ à $C_3$, éventuellement substituée par un ou plusieurs groupes OH et/ou COOH ; et
- leurs mélanges.

De préférence, l'acide est un monoacide, notamment de formule R-COOH dans laquelle R représente un atome d'hydrogène ou une chaîne alkyle, linéaire ou ramifiée, en $C_1$ à $C_5$, de préférence en $C_1$ à $C_3$, éventuellement substituée par un ou plusieurs groupes OH. De préférence, l'acide carboxylique est l'acide formique, l'acide acétique, l'acide malique, l'acide citrique, l'acide oxalique, l'acide lactique ou leurs mélanges.

De façon plus préférée, l'acide carboxylique est l'acide acétique. Cet acide présente l'avantage d'être stable dans le milieu réactionnel et d'être facilement éliminé notamment par évaporation sous vide.

De façon avantageuse, lorsque l'acide carboxylique est l'acide acétique, la conversion du pentose, par exemple xylose, de départ et la purification du furfural obtenu est facilitée du fait de la volatilité de l'acide acétique. Il sera ainsi possible d'obtenir une composition d'acide acétique riche en furfural qui pourra être directement utilisée, notamment pour la préparation de polymère, par exemple de résines furaniques ; ou de purifier cette solution pour obtenir le furfural pur.

La présence de l'acide selon l'invention, en comparaison avec le même procédé mis en oeuvre uniquement en présence d'eau, permet d'augmenter significativement la conversion du xylose, le rendement et la sélectivité en furfural. L'homme du métier, selon qu'il préfère favoriser la conversion du xylose ou la sélectivité en furfural ou avoir un bon compromis entre ces deux caractéristiques pourra déterminer la proportion et la nature de l'acide à intégrer au milieu réactionnel.

Les inventeurs ont mis en évidence que le contrôle de la concentration en acide permet de manière particulièrement avantageuse et surprenante d'obtenir un rendement et une sélectivité en HMF importants. A faible concentration en acide, le rendement en HMF est très faible voire nul et à forte concentration en acide le rendement en HMF est faible et l'acide lévullinique est obtenu en tant que sous produit en quantités importantes.

La quantité d'acide ne doit cependant pas être trop importante au risque d'augmenter la production de produits secondaires, notamment d'humine. Ainsi, de préférence la quantité d'acide est inférieure à 80 % en poids, généralement comprise entre 5 et 80 % en poids, notamment comprise entre 5 et 70 % en poids, plus préférentiellement entre 10 et 50 % en poids, par exemple 20 % ou 10 % en poids par rapport au poids total eau + acide carboxylique.

Il a de plus été montré par les inventeurs que, de façon surprenante, les acides carboxyliques, et en particulier l'acide acétique, permettaient de stabiliser le furfural formé dans le milieu aqueux.

Dans le procédé selon l'invention, l'eau est présente de préférence en une quantité supérieure ou égale à 20 % en poids, généralement en une quantité comprise entre 20 et 95 % en poids, de préférence entre 30 et 95 % en poids, par exemple entre 50 et 90 % en poids , par exemple de 80 à 90 % en poids par rapport au poids total eau + acide carboxylique.

Dans le procédé de l'invention, la quantité de xylose dépend de sa limite de solubilité dans le mélange d'eau et d'acide carboxylique. Elle est généralement comprise entre 0,5 et 10%, de préférence entre 1 % et 10 %, plus préférentiellement entre 0,5 et 5%, notamment entre 0,8 et 2%, par exemple 1 % environ, par rapport au poids total eau + acide carboxylique.

Le procédé selon l'invention peut être mené à une température comprise entre 100 et 200 °C, de préférence entre 120 et 180 °C, par exemple entre 150 et 180 °C.

Le procédé selon l'invention peut être mis en oeuvre à pression atmosphérique ou sous pression d'un gaz inerte, par exemple l'hélium, jusqu'à une pression de 3,5 MPa environ (soit 35 bars environ).

Le procédé de l'invention est mis en oeuvre en présence d'un catalyseur acide hétérogène. De façon surprenante, les inventeurs ont montré que l'association d'un acide carboxylique avec un catalyseur acide hétérogène produit un effet synergique sur la conversion des pentoses et le rendement en furfural. La synergie peut être évaluée grâce à la méthode de Colby en utilisant la formule

$$E = X + Y - (XY/100)$$

dans laquelle :

- E représente le rendement en furfural attendu en mettant en oeuvre simultanément l'acide et le catalyseur,
- X représente le rendement en furfural obtenu en mettant en oeuvre l'acide seul,
- Y représente le rendement en furfural obtenu en mettant en oeuvre le catalyseur seul. Lorsque le rendement en furfural expérimental est supérieur à E l'effet synergique est démontré.

De préférence, le catalyseur est choisi parmi les polyoxométallates (hétéropolyacides) comme l'acide 12-tungstophosphorique, de préférence dispersé sur oxyde de niobium, oxyde de zirconium, dioxyde de titane, alumine, les zircones sulfatées ; les zircones tungstées (ZrW), les sels acides de césium de l'acide 12-tungstophosphorique ($Cs_2HPW_{12}O_{40}$); les charbons et dérivés, par exemple les charbons sulfonés, les charbons fonctionnalisés, par exemple par des groupements carboxyliques, par exemple suite à une oxydation, par exemple oxydation par de l'eau de javel, les charbons imprégnés par exemple par une solution de nafion, les charbons actifs, les charbons mésoporeux, les graphites exfoliés, ou leurs mélanges, les zeolites comme la USY, Beta, MCM-22, ZSM-5, des argiles comme la Montmorillonite, éventuellement échangées par des métaux de transition, des argiles protoniques notamment de type K10, des phosphates comme les phosphates de niobium ou de Fe, des résines comme l'Amberlyst® 15 ou le Nafion®, l'oxyde de nobium ou leurs mélanges.

De préférence, le catalyseur est choisi parmi les polyoxométallates (hétéropolyacides) comme l'acide 12-tungstophosphorique, de préférence dispersé sur oxyde de niobium, oxyde de zirconium, dioxyde de titane, alumine, les zircones sulfatées ; les zircones tungstées (ZrW), les sels acides de césium de l'acide 12-tungstophosphorique ($Cs_2HPW_{12}O_{40}$); les charbons et dérivés, par exemple les charbons sulfonés, les charbons fonctionnalisés, par exemple par des groupements carboxyliques, par exemple suite à une oxydation, par exemple oxydation par de l'eau de javel, les charbons imprégnés par exemple par une solution de nafion, les charbons actifs, les charbons mésoporeux, les graphites exfoliés, ou leurs mélanges, les zeolites comme la USY, Beta, MCM-22, des argiles comme la Montmorillonite, éventuellement échangées par des métaux de transition, des phosphates comme les phosphates de niobium ou de Fe, des résines comme l'Amberlyst® 15 ou le Nafion®.

L'ajout de ces catalyseurs permet notamment de manière avantageuse d'augmenter la conversion des pentoses, par exemple le xylose, et le rendement en furfural. La sélectivité en furfural est améliorée par l'utilisation de catalyseur hétérogène

De façon particulièrement préférée le catalyseur est un charbon sulfoné, un charbon fonctionnalisé par des fonctions carboxyliques ou un charbon imprégné par des solutions d'acides carboxyliques ou autres acides ou un charbon traité par des oxydants (eau de javel, peroxydes d'hydrogène) afin accroître son acidité superficielle.

De manière plus préférée, le catalyseur est un charbon sulfoné. La quantité de catalyseur est de préférence comprise entre 2 et 100% en poids, de préférence entre 2 et 10 % en poids, par exemple 5 % environ en poids par rapport au poids de xylose.

La présence d'un catalyseur hétérogène permet avantageusement d'améliorer la sélectivité en furfural tout en diminuant la température de réaction.

De manière préférée, le procédé est mis en oeuvre en présence d'un catalyseur et à une température comprise entre 100 et 150°C, par exemple à 150°C environ.

Il est ainsi possible d'obtenir une conversion allant jusqu'à 80% des pentoses avec une sélectivité pouvant aller jusqu'à 95%.

Le procédé selon l'invention peut être mis en oeuvre en batch ou en continu. Il sera avantageusement mis en oeuvre en continu. Si un catalyseur acide hétérogène est utilisé, le procédé sera avantageusement mis en oeuvre en continu, de préférence sur un lit fixe de catalyseur.

La figure 1 représente l'influence de la température sur la conversion du xylose en furfural

La figure 2 représente l'influence de la concentration en acide acétique sur la conversion du xylose en furfural à 180°C.

La figure 3 représente l'influence de la nature de l'acide carboxylique sur la conversion du pentose en furfural à 180 °C.

La figure 4 représente l'influence de la concentration en xylose sur la conversion à 180 °C.

La figure 5 représente l'influence de la présence de catalyseur hétérogène sur la conversion du xylose en furfural à 150°C.

La figure 6 représente l'influence de la teneur en catalyseur hétérogène sur la conversion du xylose en furfural à 150°C.

Légendes dans les figures : rendt= rendement, AcAc=acide acétique, Ac=acide, (cata=catalyseur hétérogène).

La présente invention va maintenant être décrite à l'aide d'exemples non limitatifs.

Exemple 1 : Influence de la température sur la production de furfural en milieu eau-acide acétique (pas selon l'invention)

La synthèse du furfural est réalisée dans un autoclave de 100ml. Les quantités suivantes sont introduites dans le réacteur : 48 g d'eau distillée auxquels sont ajoutés 12 g d'acide acétique et 0.6g de xylose (1 %). La réaction est menée sous une atmosphère d'Helium (20 bars). Le milieu réactionnel est agité au moyen d'un agitateur magnétique. Le milieu réactionnel est porté à la température de réaction au moyen de résistances électriques régulées. Les températures suivantes sont étudiées : 120°C, 150°C, 180°C. Après 15h de réaction, le milieu réactionnel est refroidi au moyen d'un bain de glace. La conversion du xylose et le rendement en furfural sont déterminés par analyse HPLC-RID (colonne : COREGEL 87C).

Les résultats sont présentés sur la figure 1. Les résultats montrent que la conversion du xylose et le rendement en furfural sont faibles à 120°C. La figure 1 montre une augmentation de la conversion du xylose et du rendement en furfural avec l'augmentation de température. Le meilleur compromis entre la conversion du xylose et le rendement en furfural est obtenu à la température de 150°C, une selectivite de 89% en furfural est obtenue. On observe aussi qu'à la température de 180°C, la conversion du xylose est totale et le rendement molaire en furfural est proche de 80%.

Exemple 2 : Influence de la teneur en acide carboxylique (pas selon l'invention)

La synthèse de furfural est réalisée dans un autoclave de 100ml. Les quantités suivantes sont introduites dans le réacteur: 0.6g de xylose (1 %), 60 g d'une solution aqueuse pouvant contenir 10 %, 20 %, 50 % en poids d'acide acétique ou 60 g d'acide acétique. La réaction est mise en oeuvre sous une atmosphère d'hélium (20 bars). Le milieu réactionnel est agité au moyen d'un agitateur magnétique. Le milieu réactionnel est porté à la température de réaction au moyen de résistances électriques régulées à 180°C. Après 15h de réaction à 180 °C, le mélange réactionnel est refroidi au moyen d'un bain de glace. La conversion du xylose et le rendement molaire en furfural sont déterminés par analyse HPLC-RID (colonne : COREGEL 87C).

Le procédé a été mis en oeuvre avec de l'acide acétique en différentes proportions à savoir 10 %, 20 %, 50 % en poids du milieu réactionnel aqueux comprenant le xylose. Le procédé a également été mis en oeuvre avec un milieu réactionnel comprenant uniquement l'acide acétique et le xylose.

Les résultats obtenus sont présentés à la figure 2. Les résultats montrent que pour des quantités élevées d'acide carboxylique, la conversion du xylose augmente, mais la sélectivité en furfural décroît. Ainsi, lorsque la réaction est mise en oeuvre avec 100 % d'acide acétique, une quantité très faible de furfural est obtenue.

Le meilleur compromis entre conversion du xylose et sélectivité en furfural est obtenu avec une solution aqueuse comprenant 20 % d'acide acétique.

Exemple 3 : Influence de la nature de l'acide carboxylique (pas selon l'invention)

La synthèse de furfural est réalisée dans un autoclave de 100ml. Les quantités suivantes sont introduites dans le réacteur : 60 g d'une solution aqueuse contenant 20% en poids d'acide carboxylique, 0,6 g de xylose.

Les solutions aqueuses d'acide carboxylique ont les compositions suivantes :

- 12g d'acide lactique ajoutés à 48 g d'eau distillée ; ou
- 12g d'acide acétique ajoutés à 48 g d'eau distillée ; ou
- 12g d'acide formique ajoutés à 48 g d'eau distillée.

Les réactions sont menées sous une atmosphère d'hélium (20 bars). Le milieu réactionnel est agité au moyen d'un agitateur magnétique. Le milieu réactionnel est porté à la température de réaction au moyen de résistances électriques régulées à 180 °C. Après 15h de réaction à 180°C le mélange réactionnel est refroidi au moyen d'un bain de glace. La conversion du xylose et le rendement molaire en furfural sont déterminés par analyse HPLC-RID (colonne : COREGEL 87C).

Les résultats obtenus sont présentés à la figure 3. Les résultats montrent que la conversion du xylose est totale mais que le rendement en furfural est faible en présence d'acide formique et d'acide lactique. Le meilleur compromis entre conversion et rendement en furfural est obtenu avec l'acide acétique.

Exemple 4 : Influence de la teneur en xylose dans le milieu réactionnel de départ. r (pas selon l'invention)

10 La synthèse du furfural est réalisée dans un autoclave de 100ml. Les quantités suivantes sont introduites dans le réacteur : 48 g d'eau distillée et 12 g d'acide acétique auxquels sont ajoutés 0,3 g de xylose (0,5 %), ou 0,6 g de xylose (1 %), ou 3 g de xylose (5 %) ou 6 g de xylose (10 %). Les réactions sont mises en oeuvre sous une atmosphère d'hélium (20 bars). Le milieu réactionnel est agité au moyen d'un agitateur magnétique. Le milieu réactionnel est porté

à la température de réaction au moyen de résistances électriques régulées à 180°C. Après 15h de réaction à 180°C, l'autoclave est refroidi au moyen d'un bain de glace. La conversion du xylose et le rendement molaire en furfural sont déterminés par analyse HPLC-RID (colonne : COREGEL 87C).

Les résultats sont présentés sur la figure 4. Les résultats montrent que la conversion du xylose et le rendement en furfural dépendent de la concentration en xylose. Les résultats montrent que le meilleur rendement en furfural est obtenu pour 1% de xylose.

Exemple 5 : Influence de l'ajout de catalyseurs acides hétérogènes

La synthèse du furfural est réalisée dans un autoclave de 100ml. Les quantités suivantes sont introduites dans le réacteur : 48 g d'eau distillée, 12 g d'acide acétique, 0,6 g de xylose, 30 mg de catalyseur. Le catalyseur est utilisé sans prétraitement. Les réactions sont menées sous atmosphère d'hélium (20 bars). Le milieu réactionnel est agité au moyen d'un agitateur magnétique. Le milieu réactionnel est porté à la température de réaction au moyen de résistances électriques régulées à 150°C. Après 15h de réaction à 150°C le mélange réactionnel est refroidi au moyen d'un bain de glace. Le catalyseur est séparé par filtration. La conversion du xylose et le rendement molaire en furfural sont déterminés par analyse HPLC-RID (colonne : COREGEL 87C).

Le procédé a été mis en oeuvre en l'absence de catalyseur hétérogène et en présence de différents catalyseurs, à savoir le charbon sulfoné (C sulfoné), le charbon imprégné par une solution de nafion, les zircones sulfatées (ZrW), un sel acide de césium de l'acide 12-tungstophosphorique ($Cs_2H$ : $Cs_2HPW_{12}O_{40}$). Le procédé a également été mis en oeuvre en l'absence d'acide acétique (dans un milieu purement aqueux) et en présence de charbon sulfoné.

Les résultats obtenus sont présentés à la figure 5. Les résultats montrent que l'ajout de catalyseur permet d'augmenter la conversion du xylose. Les résultats montrent notamment que le meilleur compromis entre conversion du xylose et rendement en furfural est obtenu avec le charbon sulfoné utilisé dans la solution aqueuse contenant 20% en poids d'acide acétique, la sélectivité en furfural est alors proche de 95% pour une conversion du xylose supérieure à 60%.

Exemple 6 : Influence de la teneur en catalyseur

La synthèse du furfural est réalisée dans un autoclave de 100ml. Les quantités suivantes sont introduites dans le réacteur : 48 g d'eau distillée, 12 g d'acide acétique, 0,6 g de xylose et en l'absence ou en présence de catalyseur hétérogène. Les réactions sont menées sous atmosphère d'hélium (20 bars). Le milieu réactionnel est agité au moyen d'un agitateur magnétique. Le milieu réactionnel est porté à la température de réaction au moyen de résistances électriques régulées à 150°C. Après 15h de réaction à 150°C le mélange réactionnel est refroidi au moyen d'un bain de glace. Le catalyseur est séparé par filtration. La conversion du xylose et le rendement molaire en furfural sont déterminés par analyse HPLC-RID (colonne : COREGEL 87C).

Le procédé a été mis en oeuvre en l'absence de catalyseur et en présence de 30mg, 60mg ou 90mg de charbon sulfoné.

Les résultats obtenus sont présentés à la figure 6. Les résultats montrent que la teneur en catalyseur a peu d'influence sur la conversion et le rendement. Les meilleurs résultats sont obtenus pour un milieu réactionnel comprenant 5% de catalyseur par rapport au poids de xylose.

Exemple 7 : Mise en évidence de la synergie

La synthèse du furfural est réalisée dans un autoclave de 100ml. Les quantités suivantes sont introduites dans le réacteur : 48 g d'eau distillée, 12 g d'acide acétique, 0,6 g de xylose, 30 mg de catalyseur. Le catalyseur (K10, ZSM5 et Csulfoné) est utilisé sans prétraitement. Les réactions sont menées sous atmosphère d'hélium (20 bars). Le milieu réactionnel est agité au moyen d'un agitateur magnétique. Le milieu réactionnel est porté à la température de réaction au moyen de résistances électriques régulées à 150°C. Après 15h de réaction à 150°C le mélange réactionnel est refroidi au moyen d'un bain de glace. Le catalyseur est séparé par filtration. La conversion du xylose et le rendement molaire en furfural sont déterminés par analyse HPLC-RID (colonne : COREGEL 87C).

A titre de comparaison, le procédé a également été mis en oeuvre en l'absence de catalyseur mais avec 12 g d'acide acétique et en l'absence d'acide acétique et avec 30 mg de catalyseur.

Les résultats sont donnés dans le tableau 1. Le résultat en terme de rendement en furfural a été calculé par la méthode de Colby et est noté (E) dans le tableau 1.

Tableau 1

| Essais | Conditions | Conversion | Rendement Furfural | E |
|---|---|---|---|---|
| 1 | acide acétique seul | 32 | 28 | |
| 2 | K10 seul | 22 | 12 | |
| 3 | ZSM5 seul | 22 | 13 | |
| 4 | K10 + acide acétique | 62 | 40 | 36,64 |
| 5 | ZSM5 + acide acétique | 100 | 45 | 37,36 |
| 6 | 5%Csulfonée seul | 23 | 13 | |
| 7 | 5%Csulfonée + acide acétique | 61 | 56 | 37,36 |

Les résultats montrent que l'association acide carboxylique et catalyseur est synergique puisque le rendement en furfural obtenu est supérieur au rendement attendu selon la méthode de Colby.

**Revendications**

1. Procédé de préparation de furfural par réaction de xylose dans l'eau et en présence d'acide carboxylique et de catalyseur acide hétérogène, le procédé étant conduit dans une solution aqueuse d'acide carboxylique comme milieu réactionnel.

2. Procédé selon la revendication 1, pour lequel l'acide est choisi parmi :

   - les acides de formule R-COOH dans laquelle R représente un atome d'hydrogène ou une chaîne alkyle, linéaire ou ramifiée, en $C_1$ à $C_5$, éventuellement substituée par un ou plusieurs groupes OH ;
   - les acides de formule HOOC-L-COOH dans laquelle L représente une liaison ou une chaîne alkyle, linéaire ou ramifiée, en $C_1$ à $C_5$, éventuellement substituée par un ou plusieurs groupes OH et/ou COOH ; ou
   - leurs mélanges.

3. Procédé selon l'une quelconque des revendications 1 ou 2, pour lequel l'acide est l'acide acétique.

4. Procédé selon l'une quelconque des revendications 1 à 3, pour lequel le catalyseur est choisi parmi les polyoxometallates (hétéropolyacides), les zircones sulfatées ; les zircones tungstées (ZrW), les sels acides de césium de l'acide 12-tungstophosphorique ($Cs_2HPW_{12}O_{40}$); les charbons et dérivés, les zeolites, des argiles éventuellement échangées par des métaux de transition, des argiles protoniques, des phosphates, des résines, l'acide niobique ou leurs mélanges.

5. Procédé selon la revendication 4, pour lequel :

   - les polyoxometallates (hétéropolyacides) sont choisi parmi l'acide 12-tungstophosphorique éventuellement dispersé sur oxyde de niobium, oxyde de zirconium, dioxyde de titane, alumine ;
   - les charbons et dérivés sont choisis parmi les charbons sulfonés, les charbons fonctionnalisés éventuellement par des groupements carboxyliques, les charbons imprégnés éventuellement par une solution de nafion, les charbons actifs, les charbons mésoporeux, les graphites exfoliés, ou leurs mélanges,
   - les zeolites sont choisies parmi USY, Beta, MCM-22, ZSM-5 ;
   - les argiles sont choisies parmi la Montmorillonite, éventuellement échangées par des métaux de transition, des argiles protoniques ;
   - les phosphates sont choisis parmi les phosphates de niobium ou de Fe ;
   - les résines sont choisies parmi l'Amberlyst® 15 ou le Nafion®.

6. Procédé selon l'une quelconque des revendications 1 à 5, pour lequel l'acide carboxylique est présent en une quantité de 5 à 70 % en poids, par rapport au poids total eau + acide carboxylique.

7. Procédé selon l'une quelconque des revendications 1 à 5, pour lequel l'acide carboxylique est présent en une quantité de 10 à 50% en poids par rapport au poids total eau + acide carboxylique.

8. Procédé selon l'une quelconque des revendications 1 à 7, pour lequel l'eau est présente en une quantité supérieure ou égale à 20 % en poids, par rapport au poids total eau + acide carboxylique.

9. Procédé selon l'une quelconque des revendications 1 à 7, pour lequel l'eau est présente en une quantité allant de 30 à 95 % en poids par rapport au poids total eau + acide carboxylique.

10. Procédé selon l'une des quelconque des revendications 1 à 9, réalisé en présence d'un catalyseur acide hétérogène, procédé pour lequel la quantité de catalyseur est comprise entre 1 et 100 % en poids par rapport au poids de xylose.

11. Procédé selon la revendication 10, dans lequel la quantité de catalyseur est comprise entre 2 et 10% en poids par rapport au poids de xylose.

12. Procédé selon l'une quelconque des revendications 1 à 11, pour lequel la quantité de xylose est comprise entre 0,5 et 10 %en poids par rapport au poids total eau + acide carboxylique.

13. Procédé selon la revendication 12 pour lequel la quantité de xylose est comprise entre 0,5 et 5% en poids par rapport au poids total eau+acide caroxylique.

14. Procédé selon l'une quelconque des revendications 1 à 13, mené à une température comprise entre 100 et 200°C.

15. Procédé selon l'une quelconque des revendications 1 à 14, mené en continu.

**Patentansprüche**

1. Verfahren zur Herstellung von Furfural über eine Reaktion von Xylose in Wasser und in Gegenwart einer Carbonsäure und einem heterogenen Säurekatalysator, wobei das Verfahren in einer wässrigen Lösung einer Carbonsäure als Reaktionsmedium durchgeführt wird.

2. Verfahren gemäß Anspruch 1, bei dem die Säure ausgewählt ist aus:

   - Säuren der Formel R-COOH, in welcher R ein Wasserstoffatom oder eine lineare oder verzweigte $C_1$-$C_5$-Alkylkette, darstellt, die gegebenenfalls mit einer oder mehreren OH-Gruppen substituiert ist;
   - Säuren der Formel HOOC-L-COOH, in welcher L eine Bindung oder eine lineare oder verzweigte $C_1$-$C_5$-Alkylkette darstellt, die gegebenenfalls mit einer oder mehreren OH-Gruppen und/oder COOH-Gruppen substituiert ist; oder
   - Mischungen hiervon.

3. Verfahren gemäß einem der Ansprüche 1 oder 2, bei dem die Säure Essigsäure ist.

4. Verfahren gemäß einem der Ansprüche 1 bis 3, bei dem der Katalysator ausgewählt ist aus den (heteropolyaciden) Polyoxometallaten, Zirkonsulfaten, Zirkonwolframiden, sauren Cäsiumsalzen der Säure 12-Wolframatophosphorsäure ($Cs_2HPW_{12}O_{40}$), Kohlen und deren Derivate, Zeolithen, gegebenenfalls mit Übergangsmetallen ausgetauschten Tonen, protonierten Tonen, Phosphaten, Harzen, Niobsäure oder Mischungen hiervon.

5. Verfahren gemäß Anspruch 4, bei dem:

   - die (heteropolyaciden) Polyoxometallate ausgewählt sind aus 12-Wolframatophosphorsäure, die gegebenenfalls verteilt ist auf Nioboxid, Zirkonoxid, Titandioxid, Aluminium;
   - die Kohlen und Derivate ausgewählt sind aus sulfonierten Kohlen, gegebenenfalls über Carboxylgruppen funktionalisierten Kohlen, gegebenenfalls über eine Nafion-Lösung imprägnierte Kohlen, Aktivkohlen, mesoporöse Kohlen, Schichtgraphit, oder Mischungen hiervon;
   - die Zeolithe ausgewählt sind aus USY, Beta, MCM-22, ZSM-5;
   - die Tone ausgewählt sind aus gegebenenfalls mit Übergangsmetallen ausgetauschten Montmorillonit, protonierten Tonen;
   - die Phosphate ausgewählt sind aus Niobphosphat oder Eisenphosphat;
   - die Harze ausgewählt sind aus Amberlyst® 15 oder Nafion®.

**6.** Verfahren gemäß einem der Ansprüche 1 bis 5, bei dem die Carbonsäure in einer Menge von 5 bis 70 Gew.-%, gegenüber dem Gesamtgewicht aus Wasser + Carbonsäure, vorhanden ist.

**7.** Verfahren gemäß einem der Ansprüche 1 bis 5, bei dem die Carbonsäure in einer Menge von 10 bis 50 Gew.-%, gegenüber dem Gesamtgewicht aus Wasser + Carbonsäure, vorhanden ist.

**8.** Verfahren gemäß einem der Ansprüche 1 bis 7, bei dem das Wasser in einer Menge von mehr als oder gleich 20 Gew.-%, gegenüber dem Gesamtgewicht aus Wasser + Carbonsäure, vorhanden ist.

**9.** Verfahren gemäß einem der Ansprüche 1 bis 7, bei dem das Wasser in einer Menge von 30 bis 95 Gew.-%, gegenüber dem Gesamtgewicht aus Wasser+ Carbonsäure, vorhanden ist.

**10.** Verfahren gemäß einem der Ansprüche 1 bis 9, durchgeführt in Gegenwart eines heterogenen Säurekatalysators, bei welchem Verfahren die Menge an Katalysator zwischen 1 und 100 Gew.-%, gegenüber dem Gewicht von Xylose, liegt.

**11.** Verfahren gemäß Anspruch 10, bei dem die Menge an Katalysator zwischen 2 und 10 Gew.-%, gegenüber dem Gewicht von Xylose, liegt.

**12.** Verfahren gemäß einem der Ansprüche 1 bis 11, bei dem die Menge an Xylose zwischen 0,5 und 10 Gew.-%, gegenüber dem Gesamtgewicht von Wasser + Carbonsäure, liegt.

**13.** Verfahren gemäß Anspruch 12, bei dem die Menge an Xylose zwischen 0,5 und 5 Gew.-%, gegenüber dem Gesamtgewicht von Wasser + Carbonsäure, liegt.

**14.** Verfahren gemäß einem der Ansprüche 1 bis 13, durchgeführt bei einer Temperatur zwischen 100 und 200 °C.

**15.** Verfahren gemäß einem der Ansprüche 1 bis 14, kontinuierlich durchgeführt.

## Claims

**1.** A method for preparing furfural by reaction of xylose in water and in the presence of carboxylic acid and of a heterogeneous acid catalyst, the method being conducted in an aqueous solution of carboxylic acid as a reaction medium.

**2.** The method according to claim 1, for which the acid is selected from:

- the acids of formula R-COOH wherein R represents a hydrogen atom or a linear or branched $C_1$-$C_5$ alkyl chain, optionally substituted with one or several OH groups;
- acids of formula HOOC-L-COOH wherein L represents a bond or a linear or branched $C_1$-$C_5$ alkyl chain, optionally substituted with one or several OH and/or COOH groups; or
- mixtures thereof.

**3.** The method according to claim 1 or 2, for which the acid is acetic acid.

**4.** The method according to any of claims 1 to 3, for which the catalyst is selected from polyoxometallates (heteropolyacids), sulfated zirconias; tungstated zirconias (ZrW), cesium acid salts of 12-tungstophosphoric acid ($Cs_2HPW_{12}O_{40}$); coals and derivatives, zeolites , clays , optionally having been exchanged with transition metals, protonic clays, phosphates, resins, niobic acid or their mixtures.

**5.** Method according to claim 4, wherein:

- polyoxometallates (heteropolyacids) are chosen among acid 12-tungstophosphoric, optionally dispersed on niobium oxide, zirconium oxide, titanium dioxide, alumina;
- coals and derivatives chosen among sulfonated coals, optionally functionalized coals by carboxylic groups, optionally impregnated coals by nafion solution, active coals, mesoporous coals, exfoliated graphites or their mixtures;

- zeolites chosen among USY, Beta, MCM-22, ZSM-5;
- clays chosen Montmorillonite optionally having been exchanged with transition metals, protonic clays;
- phosphates chosen among niobium or Fe phosphates;
- resins chosen among Amberlyst 15 of Nafion.

**6.** The method according to any of claims 1 to 5, for which the carboxylic acid is present in an amount from 5 to 70% by weight based on the total water + carboxylic acid weight.

**7.** The method according to any of claims 1 to 5, for which the carboxylic acid is present in an amount from 10 to 50% by weight based on the total water + carboxylic acid weight.

**8.** The method according to any of claims 1 to 7, for which the water is present in an amount greater than or equal to 20% by weight, based on the total water + carboxylic acid weight.

**9.** The method according to any of claims 1 to 7, for which the water is present in an amount from 30 to 95% by weight based on the total water + carboxylic acid weight.

**10.** The method according to any of claims 1 to 9 carried out in the presence of a heterogeneous acid catalyst, a method for which the amount of catalyst is comprised between 1 and 100% by weight based on the weight of xylose.

**11.** The method according to claim 10, wherein the amount of catalyst is comprised between 2 and 10% by weight based on the weight of xylose.

**12.** The method according to any of claims 1 to 11, for which the amount of xylose is comprised between 0.5 and 10%, by weight based on the total water + carboxylic acid weight.

**13.** The method according to claim 12 for which the amount of xylose is comprised between 0,5 and 5% by weight based on the total water + carboxylic acid weight.

**14.** The method according to any of claims 1 to 13, conducted at a temperature comprised between 100 and 200°C.

**15.** The method according to any of claims 1 to 14, conducted continuously.

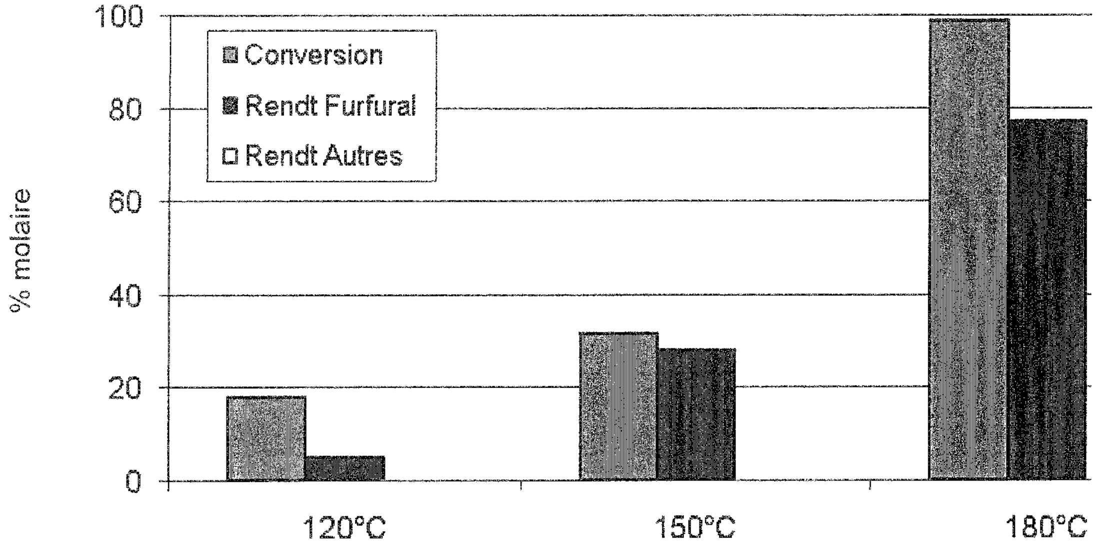
100
80
60
40
20
0
% molaire
Conversion
Rendt Furfural
Rendt Autres
120°C
150°C
180°C

Fig. 1

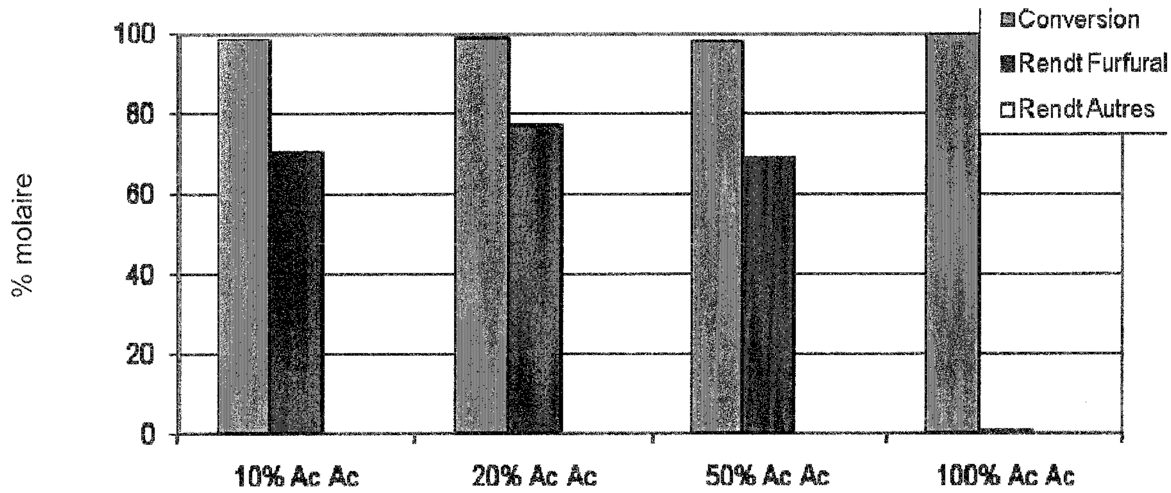
100
80
60
40
20
0
% molaire
Conversion
Rendt Furfural
Rendt Autres
10% Ac Ac
20% Ac Ac
50% Ac Ac
100% Ac Ac

Fig. 2

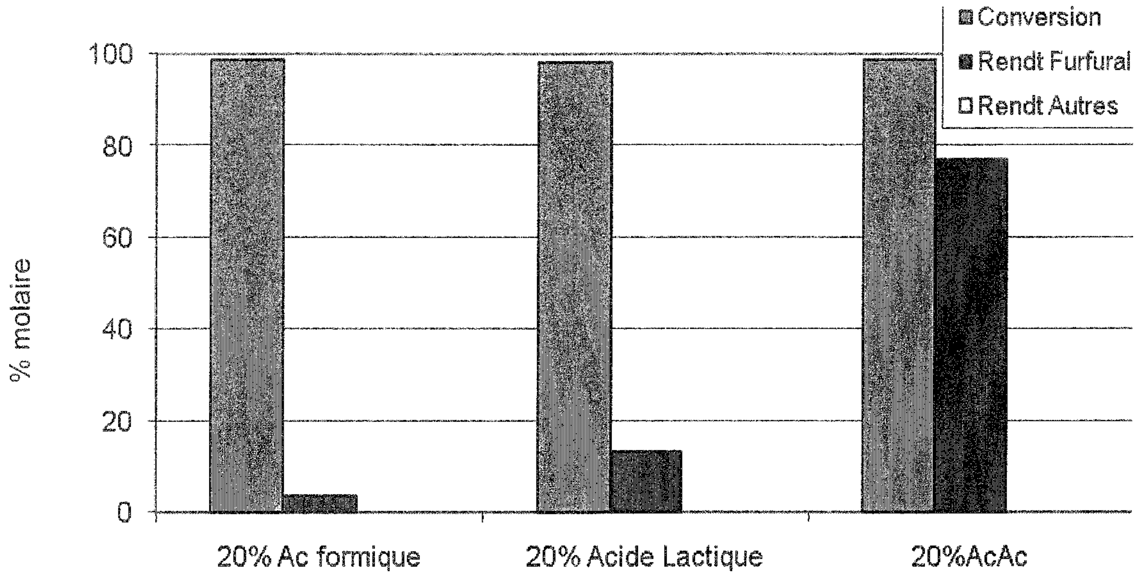
Conversion
Rendt Furfural
Rendt Autres
% molaire
100
80
60
40
20
0
20% Ac formique
20% Acide Lactique
20%AcAc

Fig. 3

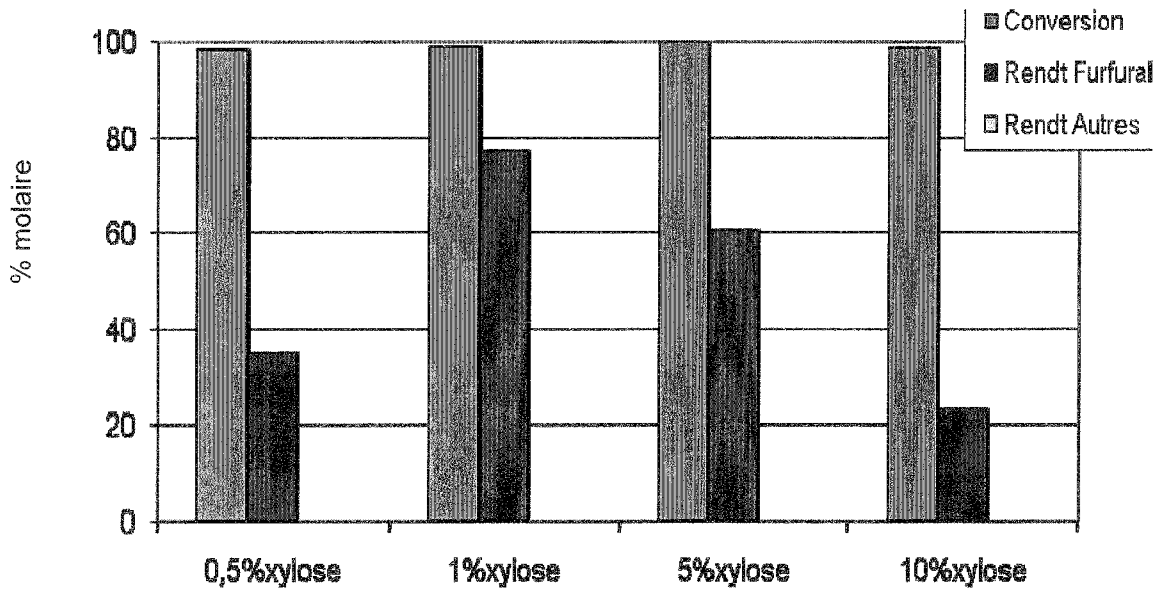
Conversion
Rendt Furfural
Rendt Autres
% molaire
100
80
60
40
20
0
0,5%xylose
1%xylose
5%xylose
10%xylose

Fig. 4

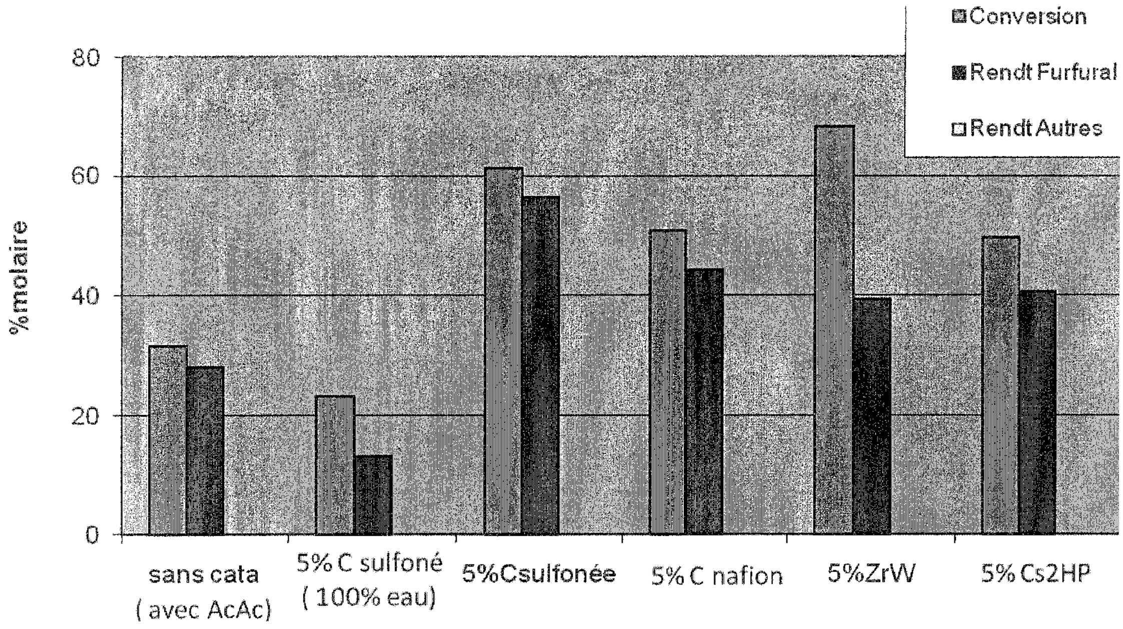
Conversion
Rendt Furfural
Rendt Autres
80
60
40
20
0
%molaire
sans cata
( avec AcAc)
5% C sulfoné
( 100% eau)
5%Csulfonée
5% C nafion
5%ZrW
5% Cs2HP

Fig. 5

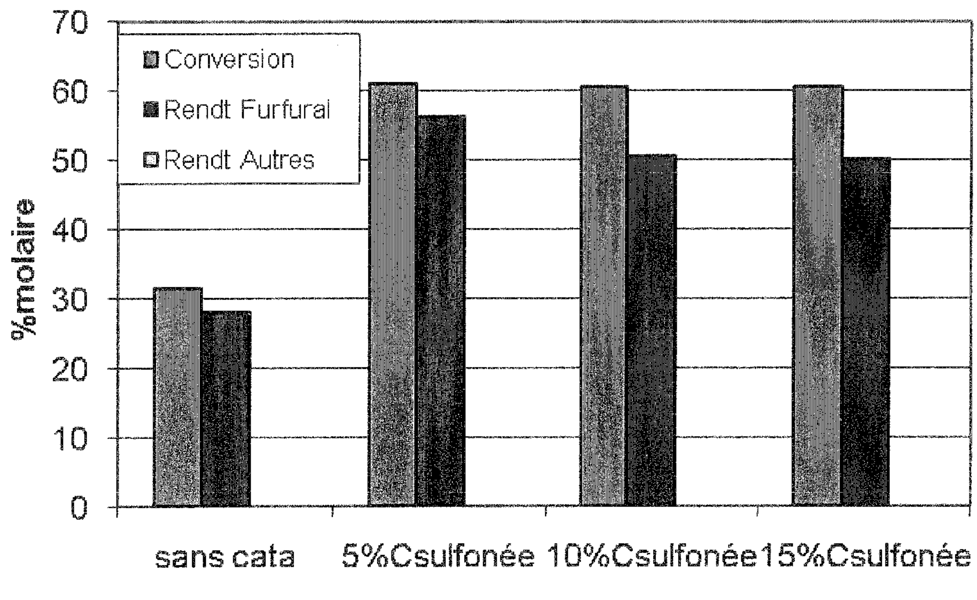
70
60
50
40
30
20
10
0
%molaire
Conversion
Rendt Furfural
Rendt Autres
sans cata
5%Csulfonée
10%Csulfonée
15%Csulfonée

Fig. 6

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2007146636 A


### Littérature non-brevet citée dans la description


- **MOREAU et al.** *Industrial Crops and Products,* 1998, vol. 7, 95-99
- **LIMA et al.** *Applied Catalysis A : General,* 2010, vol. 388, 141-148
- **WANG et al.** *Energies,* 2011, 670-684
- **HUBERT et al.** *Journal of Catalysis,* 2011, vol. 279, 174-182
- **HUBERT et al.** *Green Chemistry,* 2010, vol. 12, 1423-1429